# FASCICULE DE BREVET EUROPEEN

(11) **EP 0 586 275 B1**
(45) Date de publication et mention de la délivrance du brevet: **08.10.1997**
(21) Numéro de dépôt: 93401980.3
(22) Date de dépôt: 30.07.1993
(51) Int. Cl.: A61K 7/00, A61K 7/08, A61K 7/50, A61K 7/48

(54) **Composition cosmétique contenant en suspension des particules non hydrosolubles**
Kosmetische Zusammensetzung die unwasserlösliche Partikel in Form einer Suspension enthält
Cosmetic composition containing water-insoluble particles in suspension

(30) Priorité: 05.08.1992 FR 9209692
(43) Date de publication de la demande: 09.03.1994
(73) Titulaire: RHONE-POULENC CHIMIE, 92408 Courbevoie Cédex (FR)
(72) Inventeur: Derian, Paul-Joel, F-92260 - Fontenay-Aux-Roses (FR); Willemin, Claudie, F-75018 Paris (FR)
(74) Mandataire: Seugnet, Jean Louis

(56) Documents cités:
- EP-A- 0 043 327
- EP-A- 0 091 331
- EP-A- 0 210 774
- EP-A- 0 285 389
- WO-A-92/01507
- GB-A- 2 188 058
- US-A- 4 606 913

## Description

La présente invention a pour objet une formulation cosmétique fluide, contenant en suspension, de manière homogène et stabilisée, des particules non hydrosolubles.

Généralement, les formulations de produits cosmétiques de type shampooings, gels douches ou lotions démaquillantes incorporent une base lavante, éventuellement un agent moussant, des agents tensio-actifs et divers additifs de type parfum ou colorant. Certaines formulations plus sophistiquées contiennent également des matières actives.

Tant que ces matières actives demeurent hydrosolubles, la mise au point des formulations cosmétiques, capillaires ou corporelles, correspondantes ne pose pas de difficultés quelconques. En revanche, l'incorporation de matières actives non hydrosolubles soulèvent des problèmes de stabilité. Il est impératif d'obtenir des formulations homogènes et qui ne conduisent pas au cours d'un stockage prolongé à une sédimentation ou une démixtion de phase.

La solution actuellement préconisée pour éviter ce problème consiste à incorporer un épaississant dans la formulation cosmétique. Généralement, il s'agit d'un hydrocolloïde comme la gomme xanthane par exemple.

L'utilisation de ce type de composés épaississants ne donne toutefois pas toujours satisfaction. En particulier, l'aspect des formulations cosmétiques correspondantes peut ne pas répondre aux exigences cosmétiques. Elles présentent généralement un aspect glaireux ou filant et en conséquence peu adapté pour un écoulement fluide lisse et homogène, particulièrement apprécié de l'utilisateur. Enfin, les hydrocolloïdes sont des composés coûteux qui accroissent le prix de revient des formulations les incorporant.

La demande de brevet GB-A-2 188 058 décrit une composition pour le lavage des mains à base de particules solides dispersées dans une phase pseudoplastique constituée d'un agent tensioactif anionique, d'un agent tensioactif non-ionique de HLB (hydrophile-lipophile balance) entre 10 et 15, d'un agent tensioactif de HLB entre 6 et 10, d'un solvant hydrocarboné et éventuellement d'un électrolyte.

La demande de brevet WO-A-92/01507 décrit une dispersion aqueuse stable comprenant un ester partiel de glycérol et d'un acide gras, un agent tensioactif non-ionique oxyéthyléné, un composé huileux insoluble et un électrolyte.

La présente invention a précisément pour objet de proposer des formulations cosmétiques appropriées à la mise en suspension de particules non hydrosolubles, ne nécessitant pas la mise en oeuvre de ce type d'agents épaississants et ne possédant pas en outre un comportement visqueux.

Plus particulièrement la présente invention se rapporte à une composition cosmétique fluide aqueuse contenant en suspension des particules non hydrosolubles caractérisée en ce qu'elle est exempte de solvant organique et comprend en outre au moins un tensio actif anionique, un cotensio-actif de nature non ionique ou amphotère et un électrolyte, lesdits tensio-actifs étant en quantités telles que ladite composition possède un comportement pseudoplastique avec un seuil d'écoulement égal ou supérieur à 0,2Pa et présente une structure de phase lamellaire incluant des sphérulites aptes à maintenir en suspension de manière stable et homogène lesdites particules, la teneur en base lavante étant inférieure à 30%.

La Demanderesse a constaté que l'association d'agents tensio-actifs particuliers conduit à un milieu possédant un comportement rhéologique inattendu car tout particulièrement approprié de part sa structure de phase à la mise en suspension des particules précitées.

Parmi les tensio-actifs anioniques convenant à l'invention, on peut plus particulièrement citer les sels d'acides gras ; les sels de mono- ou dialkylsulfates, mono- ou di- alkyléthersulfates, lauryléthersulfates, alkysulfonates, alkylarylsulfonates, alpha-oléfinsulfonates, alkylphosphates alcalins, les sulfonates d'ethers alkylphénolpolyglycoliques ; les sels d'esters d'acides alkylsulfopolycarboxyliques; les sels d'alkylsulfosuocinates, alkyléthersulfosuccinates, alkyliséthionates, alkyltaurates ; les produits de condensation des acides gras avec les acides oxy-et amino-alcanesulfoniques ; les dérivés sulfatés d'acide gras et de polyglycols ; les alcanolamides d'acides gras sulfatés et les sels de lipoaminoacides. Les lipoaminoacides sont obtenus par acylation des acides aminés issus de l'hydrolyse totale d'une protéine naturelle. A titre de sels, on peut plus particulièrement mentionner leurs sels de sodium, d'ammonium de magnésium et de triéthylamine.

En ce qui concerne le lauryléthersulfate de sodium LESNa, il s'agit d'un tensioactif largement employé dans les shampooings pour ses propriétés moussantes. C'est une base lavante classique de bon marché.

A titre de tensio-actif non ionique, on peut plus particulièrement citer les alcools gras éthoxylés et notamment ceux dérivant des alcools laurique, cérylstéarylique, stéarylique, cétylique et oléocétylique. On peut également employer les sucroglycérides.

Les sucroglycérides sont des mélanges de produits, obtenus par transestérification de triglycérides, naturels ou de synthèse, avec le saccharose. Ces mélanges contiennent des monoglycérides, des diglycérides, de faibles quantités de triglycérides non transestérifiés, des monoesters et diesters du saccharose.

Le brevet EP-A-0 091 331, qui décrit un procédé de préparation de sucroglycérides fluides, indique également que lesdits sucroglycérides ont des propriétés tensio-actives.

Quant aux co-tensio-actifs amphotères, ils peuvent être choisis parmi les bétaïnes comme l'oléylbétaïne et les sultaines du type cocoamidopropylhydroxysultaine et les dérivés de l'imidazoline tel que le cocoamphodiacétate.

Ces amphotères possèdent en outre l'avantage de constituer d'excellentes bases lavantes fournissant une mousse lavante, abondante et stable. Ils sont doués d'une excellente innocuité corporelle et oculaire.

A titre d'électrolyte, il est possible d'employer du chlorure de sodium ou d'ammonium.

De manière surprenante, leur association permet de conduire à une formulation aqueuse dotée de propriétés suspensives améliorées et apte à maintenir en suspension des particules non hydrosolubles solides ou non. Dans les conditions selon l'invention, on note, au cours du temps, aucune sédimentation ou démixtion quelconque des compositions cosmétiques correspondantes.

Le seuil d'écoulement de ces compositions est égal ou supérieur à 0,2Pa.. Pour des formulations à base de mélanges de tensio actifs préparés à une température de l'ordre de 50°C, ce seuil d'écoulement possède une valeur proche de 1 Pa..

Les formulations cosmétiques correspondantes présentent une structure de phase dite lamellaire comportant en suspension des sphérulites.

Une phase lamellaire désigne une phase solide hydratée ou de cristaux liquides dans laquelle plusieurs bicouches sont disposées en un réseau parallèle, séparées par des couches d'eau ou d'une solution aqueuse. Quant aux sphérulites, il s'agit de vésicules plurilamellaires constituées de plusieurs couches de tensio-actifs disposées concentriquement et de dimensions comprises généralement entre 0,1 et 50 µm.

Plus précisément, on peut définir la structure de phase correspondante comme celle d'une solution biréfringente, optiquement caractéristique d'une phase mésomorphe non isotrope.

Par le terme particules non hydrosolubles, on entend désigner selon l'invention des entités, solides ou non, qui ne se solubilisent pas dans le milieu aqueux de la composition revendiquée.

Il s'agit plus particulièrement de particules solides ou de gouttelettes émulsifiées.

A titre de particules solides, on peut citer des matières actives du type celles utilisées pour les traitements capillaires comme le zinc pyrithione ou des matières abrasives quelconques qui peuvent être d'origine naturelle ou synthétique. Il peut en particulier s'agir de polycarbonates, polypropylènes, polyéthylènes, alumine, calcite et d'argiles. Ces particules possèdent généralement une granulométrie comprise entre environ 1 et 600 µm et de préférence entre environ 10 et 400 µm.

En ce qui concerne les gouttelettes émulsifiées, il s'agit de préférence de gouttelettes d'au moins une huile végétale, huile essentielle et/ou plus particulièrement huile de silicone.

Les huiles de silicones susceptibles d'être utilisées selon l'invention peuvent être choisies parmi les polyalkylsiloxanes, les polyalkylarylsiloxanes et leurs mélanges. Les polyalkylsiloxanes appropriées sont notamment les polydiméthylsiloxanes comme la diméthicone dont la viscosité peut varier d'environ 20mPa.s à 50 Pa.s à 25°C, pure ou en mélange avec la cyclométhicone . A titre d'exemple de polyalkylarylsiloxanes on peut plus particulièrement citer les polyphényldiméthylsiloxanes.

En particulier, les polydiorganosiloxanes tels que les polydimétylsiloxanes possédant un poids moléculaire inférieur ou égal à 3 000 000 et les polydiméthyldiphénylsiloxanes de poids moléculaire proche de 600 000, peuvent être avantageusement utilisés selon l'invention.

La taille des goutellettes d'huile au sein de la composition revendiquée varient environ entre 0,5 et 50 µm.

Les formulations selon l'invention peuvent contenir approximativement de 0,5 à 8% et de préférence environ de 1,5 à 4,5% en poids de particules non hydrosolubles.

A titre de formulations particulières de l'invention on peut citer celles
- contenant à titre d'agent anionique le LESNa, de cotensio-actif l'alcool laurique à deux moles d'oxyde d'éthylène et d'électrolyte du NaCl dans un rapport molaire alcool laurique 2OE/LESNa compris entre 2,1 et 4,3,
- celles contenant à titre d'agent anionique un lipoaminoacidesodique, le lipoprotéol LCO® en mélange avec le même alcool laurique à deux motifs éthoxy et possédant un rapport molaire alcool laurique 2OE/ lipoaminoacidesodique variant environ entre 1,2 et 6,7 et
- celles contenant à titre d'agent anionique le LESNa, de cotensio-actif l'oléylbétaïne et d'électrolyte du NaCl dans un rapport molaire oléylbétaïne/ LESNa compris entre 1,1 et 3,7.

De manière générale, les compositions selon l'invention contiennent un tensio-actif anionique en mélange avec au moins un co-tensio actif dans un rapport molaire co-tensio-actif/tensio-actif anionique égal ou supérieur à 1.

L'électrolyte mis en oeuvre dans ces formulations est généralement du chlorure de sodium. Sa concentration est ajustée de manière à conduire à la formulation recherchée. De manière générale, elle est supérieure à 10g/l et de préférence comprise entre 10 et 40g/l.

Les compositions selon l'invention ne comportent pas de solvants organiques, en particulier de solvants oaganiques hydrocarbonés, qui, comme enseigné par GB 2 188 058, servent à mettre en suspension les particules solides.

Toutes les formulations correspondantes conduisent à des compositions selon l'invention c'est à dire capables de maintenir en suspension au cours d'un stockage de manière prolongée des particules non hydrosolubles et ne nécessitant pas l'incorporation d'un épaississant quelconque.

Leur teneur en base lavante est inférieure à 30%. Elles possèdent de bons pouvoirs mouillant et moussant. Enfin, elles présentent une bonne tolérance cutanée et oculaire.

Bien entendu, la composition selon l'invention peut incorporer en outre d'autres additifs de type parfums, colorants et agents conservateurs.

Les compositions selon l'invention peuvent se présenter indifféremment sous la forme de shampooings, gels douche, lotions capillaires ou gels exfolliants.

La présente invention se rapporte également à un procédé de préparation de la composition cosmétique.

Plus précisément, le procédé selon l'invention comprend:
- le mélange sous agitation, en milieu aqueux, d'au moins un tensio actif anionique, un co-tensio-actif non ionique et/ou amphotère et un électrolyte, lesdits tensio-actifs étant en quantités telles que le mélange aqueux résultant possède un comportement pseudoplastique avec un seuil d'écoulement supérieur ou égal à 0,2Pa et présente une structure de phase lamellaire incluant des sphérulites,
- l'addition audit mélange de particules solides ou d'au moins une huile avec dans le cas particulier de l'huile la mise sous très forte agitation du mélange aqueux résultant, de manière à cisailler l'huile en fines gouttelettes au sein du mélange aqueux et
- la récupération de la composition cosmétique attendue.

Le procédé selon l'invention a pour avantage, d'une part de ne pas nécessiter la préparation préalable d'une émulsion aqueuse préformée d'huile et, d'autre part, de ne pas utiliser de préfèrence, de solvants organiques, en particulier de solvants organiques hydrocarbonés, qui, comme enseigné par GB 2 188 058, servent à mettre en suspension les particules solides.
L'émulsion d'huile est formée intrinsèquement au sein du mélange aqueux. Elle y est en outre stabilisée dès sa formation.

Les exemples figurant ci-après permettront de mettre en évidence d'autres avantages et caractéristiques de la présente invention.

### EXEMPLE 1

### Mise au point de formulations appropriées à la stabilisation de particules hydrosolubles.

Les formulations présentées ci-après ont été préparées selon le protocole suivant:

Dans de l'eau salée, on incorpore le tensio-actif anionique. Le mélange est effectué sous agitation assez forte à l'aide d'un pâle-cadre a environ 50°C. Le co-tensio-actif est ensuite ajouté progressivement et l'ensemble maintenu sous agitation de manière à obtenir la structure de phase attendue qui est contrôlée au microscope optique ou électronique.

### 1.système tensio-actif A: un aaent anionique et un agent non ionique.

- L'agent anionique choisi est un lipoaminoacidesodique. Il s'agit plus particulièrement du lipoprotéol LCO® qui correspond à une solution aqueuse de sels mixtes de sodium et de triéthanolamine obtenue par combinaison d'acide laurique avec les acides aminés issus de l'hydrolyse du collagène.
- L'agent non ionique est l'alcool laurique éthoxylé à 2 moles d'oxyde d'éthylène.
- L'électrolyte est une solution aqueuse de chlorure de sodium à 16g/l.
Les formulations, impliquant ce système de tensio-actifs et appropriées à la stabilisation de particules non hydrosolubles, correspondent à une valeur du rapport molaire P **comprise entre 1,2 et 6,7, P désignant le rapport molaire** **du pourcentage molaire de tensio-actif non ionique rapporté à la valeur du** **pourcentage molaire de tensio-actif anionique.**
Ces formulations se caractérisent par une structure de phase lamellaire comportant une suspension de sphérulites. La taille des sphérulites est déterminée au microscope optique ou électronique. Elle est de l'ordre de 0,1 à 12 microns.

### 2.Dans un second système tensio-actif anionique et co tensio-actif non ionique B il est mis en oeuvre le LESNa au lieu du lipoprotéol LCO.

Il s'agit d'une solution aqueuse à 27% de lauryléthersulfate de sodium LESNa.

Pour ce système, les formulations correspondant à une suspension sphérulitique dans une phase lamellaire sont celles possédant un rapport molaire alcool laurique éthoxyl**é 20E/LESNa compris entre 2,1 et 4,3.**

### 3.Système tensio-actif: tensio-actif anionique/co tensio-actif amphotère.

L'agent anionique est le LESNa.
L'agent amphotère est une bétaïne. Il s'agit de l'oléylbétaïne.
L'électrolyte est une solution de chlorure de sodium à 40g/l.
Les formulations correspondantes appropriées à la stabilisation de particules solides c'est à dire présentant une suspension de sphérulites au sein d'une phase lamellaire ont été déterminées **pour un rapport molaire oléylbétaïne / LESNa compris approximativement entre 1,1 et 3,7**.
Le tableau I ci-après rend compte de trois formulations correspondantes.

**TABLEAU I**

| FORMULATION | A | B | C |
|---|---|---|---|
| LESNa | 5 | 5 | 4 |
| Oléylbétaïne | 5,6 | 7,7 | 7,8 |
| eau salée | 88,4 | 86 | 86,8 |

Chacune de ces trois formulations a été testée pour ses propriétés rhéologiques par la mesure de son seuil d'écoulement et caractérisés par ses propriétés viscoélastiques.

### 1) le seuil d'écoulement

Les mesures de seuil d'écoulement ont été réalisées sur les formulations A, B et C préparées à 50°C , avec un Rhéomat 30 en utilisant le modèle de Bingham.

**TABLEAU II**

| Formulation | Seuil (Pa) |
|---|---|
| A | 1,3 |
| B | 1,7 |
| C | 1,5 |

Les valeurs élevées de seuil d'écoulement témoignent des bonnes propriétés dispersantes des formulations A, B et C.

### 2) Mesure en oscillation des propriétés viscoélastiques

Elles ont été réalisées sur un spectromètre rhéométrique, RFS. Une déformation sinusoïdale est imposée au système et on mesure le couple résultant.
Les valeurs G', G'', n* et Tgte delta sont déduites de cette mesure.
G'= module élastique
G''= module viscoélastique
n*= viscoélasticité complexe
Tgte delta= G''/G'

Pour un échantillon donné, la première opération consiste à déterminer l'amplitude de déformation au dessous de laquelle G'' et G' restent constants lorsque l'on prolonge la mesure. La fréquence est fixée à 1 radian par seconde.

Dans un second temps on fixe la déformation et on effectue un balayage en fréquence, la déformation choisie se situant au niveau du plateau d'élasticité.

Pour les trois formulations sélectionnées, on enregistre un comportement viscoélastique. Pour ces trois milieux, le module G' est supérieur à G'' sur toute la gamme de fréquences comprises entre 0,1 et 100 rad/s. Ce résultat indique que les solutions sont élastiques. D'autre part, elles sont pseudoplastiques puisque la viscosité apparente est une fonction décroissante de la fréquence et de la vitesse.

### EXEMPLE 2

### Formulations de compositions cosmétiques selon l'invention

Des shampooings antipelliculaires ont été préparés à partir des trois formulations A, B et C. Les shampooings correspondants sont désignés ci-après V, W et Z.
Pour ce faire, on introduit dans chacune des trois formulations 2% d'une suspension de zinc pyrithione à 48%.
Les shampooings correspondants ont été testés pour la stabilité de leurs suspensions de zinc pyrithione, pour leur pouvoir moussant et leur pouvoir mouillant.

### 3) stabilité des suspensions de zinc pyrithione

Quelque soit la température, 20°C ou 40°C, les trois shampooings sont stables plus de trois mois.

### 4) Mesure du pouvoir moussant

Il a été mesuré selon la norme AFNOR NFT 73404 utilisée pour les tensio-actifs.
La méthode consiste à mesurer le volume et la hauteur de la mousse formée, respectivement à 30 secondes, 3 minutes et 5 minutes après la fin de l'écoulement.

**TABLEAU III**

| Formulation | 30 secondes ml | 3 minutes ml | 5 minutes ml |
|---|---|---|---|
| V | 390 | 395 | 370 |
| W | 365 | 355 | 350 |
| Z | 355 | 345 | 355 |

Ces résultats témoignent du bon pouvoir moussant des shampooings selon l'invention.

### 5) Mesure du pouvoir mouillant

Il a été mesuré selon la norme AFNOR NFT 73406. Cette méthode consiste à déterminer la concentration en grammes par litre de la solution de tensio-actif/eau distillée suffisante pour mouiller une rondelle de coton en 100 secondes, à 20°C. A titre de valeurs témoins, les pouvoirs mouillants du LESNa et de l'oléylbétaïne sont également présentées dans le tableau suivant:

**TABLEAU IV**

| Formulation | P.mouillant g/l |
|---|---|
| LESNa | 2,10 |
| oléylbétaïne | 0,8 |
| V | 0,75 |
| W | 0,80 |
| Z | 0,85 |

De manière inattendue, on n'observe aucune diminution du pouvoir mouillant des formulations selon l'invention incorporant du LESNa comparativement à la formulation témoin ne contenant que de l'oléylbétaïne. L'association de l'oléylbétaïne au LESNa permet d'augmenter de manière significative et de façon inattendue les performances de mouillage du LESNa.

### 6) mise au point d'une formulation particulière de shampooing

| | % en poids |
|---|---|
| Lauryléther sulfate de sodium | 19,2 |
| oléylbétaine | 18,8 |
| NaCl | 2,6 |
| colorant | q.s |
| Rhodialux S® (filtre UV) | 0,05 |
| parfum | q.s |
| Zn PYRION® (40%) | 2,4 |
| eau | q.s. pour 100 |

### EXEMPLE 3.

### Préparation d'une formulation de shampooing incorporant en suspension des gouttelettes d'une huile de silicone émulsifiée.

| | % en poids |
|---|---|
| Lauryléthersulfate de sodium | 26,92 |
| Oleylbétaïne | 28,03 |
| SILBIONE 71631® (Solution à 30% de polydiméthicone de viscosité 500 000 mPa.s dans cyclométhicone) | 3,33 |
| colorant-conservateur | q.s. |
| Parfum | 1,00 |
| NaCl | 1,74 |
| Eau désionisée | qsp 100 |
| Acide citrique | qsp |

Dans de l'eau salée contenant en outre un conservateur, on incorpore du lauryléthersulfate de sodium. Le mélange tensio-actif et phase aqueuse est effectué sous agitation assez forte à l'aide d'une pâle cadre à environ 50°C. L'oléylbétaïne y est à son tour ajoutée progressivement. On introduit ensuite sous très fort cisaillement l'huile de silicone en mélange avec le parfum. Le taux de cisaillement est maintenu pendant 5 à 10 minutes à 13000rpm (ultra-turrax)®. L'huile de silicone est de cette manière émulsifiée au sein de la composition. Le pH est ensuite ajusté à 6 par ajout d'acide citrique. La viscosité du mélange final est évaluée par mesure au Broockfield®. Elle est de l'ordre de 11,3 Pa.s.

## Revendications

1. Composition cosmétique fluide aqueuse, contenant en suspension des particules non hydrosolubles caractérisée en ce qu'elle est exempte de solvant organique et comprend en outre au moins un tensio actif anionique, un cotensio-actif de nature non ionique ou amphotère et un électrolyte, lesdits tensio actifs étant en quantités telles que ladite composition possède un comportement pseudoplastique avec un seuil d'écoulement égal ou supérieur à 0,2Pa et présente une structure de phase lamellaire incluant des sphérulites aptes à maintenir en suspension de manière stable et homogène lesdites particules, la teneur en base lavante étant inférieure à 30%.

2. Composition cosmétique selon la revendication 1 caractérisée en ce que le tensio-actif anionique est de préférence choisi parmi les sels d'acides gras ; les sels de mono- ou di-alkylsulfates, mono- ou di- alkyléthersulfates, lauryléthersulfates, alkylsulfonates, alkylarylsulfonates, alpha-oléfinsulfonates, alkylphosphates alcalins, les sulfonates d'ethers alkylphénolpolyglycoliques ; les sels d'esters d'acides alkylsulfopolycarboxyliques, les sels d' alkylsulfosuccinates, alkyléthersulfosuccinates, alkyliséthionates, alkyltaurates ; les produits de condensation des acides gras avec les acides oxy-et amino-alcanesulfoniques ; les dérivés sulfatés d'acide gras et de polyglycols ; les alcanolamides d'acides gras sulfatés et les sels de lipoaminoacides.

3. Composition cosmétique selon la revendication 1 ou 2 caractérisée en ce que le co-tensio-actif non ionique est de préférence choisi parmi les sucroglycérides et les alcools gras éthoxylés et de préférence parmi ceux dérivant des alcools laurique, cétylstéarylique, stéarylique, cétylique et oléocétylique.

4. Composition cosmétique selon la revendication 1,2 ou 3 caractérisée en ce que le co-tensio-actif amphotère est choisi parmi les bétaïnes comme l'oléylbétaïne et les sultaines de type cocoamidopropylhydroxysultaine et les dérivés de l'imidazoline tel que le cocoamphodiacétate.

5. Composition cosmétique selon l'une quelconque des revendications précédentes caractérisée en ce que l'électrolyte est de préférence du chlorure de sodium ou du chlorure d'ammonium.

6. Composition cosmétique selon l'une quelconque des revendications précédentes caractérisée en ce que les particules non hydrosolubles sont des particules solides et de préférence du zinc pyrithione ou des matières abrasives naturelles ou synthétiques.

7. Composition cosmétique selon l'une des revendications 1 à 5 caractérisée en ce que les particules non hydrosolubles sont des gouttelettes émulsifiées d'au moins une huile végétale, huile essentielle, et/ou huile de silicone.

8. Composition cosmétique selon la revendication 6 caractérisée en ce que la taille des particules solides est compose entre environ 1 et 600 µm et de préférence entre 10 et 400 µm.

9. Composition cosmétique selon la revendication 7 caractérisée en ce que la taille des goutellettes émulsifiées varie entre 0,5 et 50 µm.

10. Composition cosmétique selon la revendication 7 ou 9 caractérisée en ce qu'il s'agit d'une huile de silicone choisie de préférence parmi les polyalkylsiloxanes, les polyalkylarylsiloxanes et leurs mélanges.

11. Composition cosmétique selon l'une quelconque des revendications de 1 à 10 caractérisée en ce qu'elle contient environ de 0,5 % à 8 % et de préférence entre 1,5 à 4,5% de particules non hydrosolubles.

12. Composition cosmétique selon l'une quelconque des revendications précédentes caractérisée en ce qu'elle contient à titre d'agent anionique le LESNa, de cotensio-actif l'alcool laurique à deux moles d'oxyde d'éthylène et d'électrolyte du NaCl dans un rapport molaire alcool laurique 2OE/LESNa compris entre 2,1 et 4,3.

13. Composition cosmétique selon l'une des revendications 1 à 11 caractérisée en ce qu'elle contient à titre d'agent anionique le LESNa, de cotensio-actif l'oléylbétaïne et d'électrolyte du NaCl dans un rapport molaire oléylbétaïne/LESNa compris entre 1,1 et 3,7.

14. Composition cosmétique selon l'une des revendications 1 à 11 caractérisée en ce qu'elle contient à titre d'agent anionique un lipoaminoacidesodique, à titre d'agent non ionique un alcool laurique à deux motifs éthoxy dans un rapport molaire alcool laurique 2OE/ lipoaminoacidesodique variant environ entre 1,2 et 6,7.

15. Composition cosmétique selon l'une quelconque des revendications précédentes caractérisée en ce qu'il s'agit d'un shampooing, d'un gel douche, d'une lotion capillaire ou d'un gel exfolliant.

16. Composition cosmétique selon l'une quelconque des revendications précédentes caractérisée en ce qu'elle ne comporte pas de solvants organiques, en particulier de solvants hydrocarbonés

17. Procédé de préparation d'une composition cosmétique selon l'une des revendications 1 à 16 caractérisé en ce qu'il comprend:
- le mélange sous agitation en milieu aqueux d'au moins un tensio actif anionique, un co-tensio-actif non ionique et/ou amphotère et un électrolyte, lesdits tensio-actifs étant en quantités telles que le mélange aqueux résultant possède un comportement pseudoplastique avec un seuil d'écoulement supérieur ou égal à 0,2Pa et présente une structure de phase lamellaire incluant en suspension des sphérulites,
- l'addition audit mélange de particules solides ou d'au moins une huile avec dans le cas de l'huile la mise sous très forte agitation du mélange aqueux résultant de manière à émulsifier l'huile en fines gouttelettes au sein du mélange aqueux et
- la récupération de ladite composition.

## Claims

1. Aqueous free-flowing cosmetic composition containing water-insoluble particles in suspension, characterised in that it is free from organic solvent and comprises, in addition, at least one anionic surface-active agent, one cosurface-active agent of non-ionic or amphoteric nature and one electrolyte, the said surface-active agents being in amounts such that the said composition has a pseudoplastic behaviour with a yield point equal to or greater than 0.2 Pa and exhibits a lamellar phase structure enclosing spherulites capable of maintaining the said particles in suspension in a stable and homogeneous manner, the content of washing base being less than 30%.

2. Cosmetic composition according to Claim 1, characterised in that the anionic surface-active agent is preferably chosen from salts of fatty acids; alkali metal salts of mono- or di-alkyl sulphates, mono- or di-alkyl ether sulphates, lauryl ether sulphates, alkyl sulphonates, alkyl aryl sulphonates, alpha-olefinsulphonates or alkyl phosphates, sulphonates of alkylphenolpolyglycol ethers; salts of alkyl sulphopolycarboxylic acid esters; salts of alkyl sulphosuccinates, alkyl ether sulphosuccinates, alkyl isethionates or alkyl taurates; products of condensation of fatty acids with oxy- and amino-alkanesulphonic acids; sulphated derivatives of fatty acids and polyglycols; alkanolamides of sulphated fatty acids and salts of lipoamino acids.

3. Cosmetic composition according to Claim 1 or 2, characterised in that the non-ionic cosurface-active agent is preferably chosen from sucroglycerides and ethoxylated fatty alcohols and preferably from those which are derived from lauryl, cetylstearyl, stearyl, cetyl or oleocetyl alcohols.

4. Cosmetic composition according to Claim 1, 2 or 3, characterised in that the amphoteric cosurface-active agent is chosen from betaines such as oleylbetaine and sultaines of the cocoamidopropylhydroxysultaine type and imadazoline derivatives such as cocoamphodiacetate.

5. Cosmetic composition according to any one of the preceding claims, characterised in that the electrolyte is preferably sodium chloride or ammonium chloride.

6. Cosmetic composition according to any one of the preceding claims, characterised in that the water-insoluble particles are solid particles and preferably zinc pyrithione or natural or synthetic abrasive materials.

7. Cosmetic composition according to one of Claims 1 to 5, characterised in that the water-insoluble particles are emulsified droplets of at least one vegetable oil, essential oil and/or silicone oil.

8. Cosmetic composition according to Claim 6, characterised in that the size of the solid particles is between about 1 and 600 µm and preferably between 10 and 400 µm.

9. Cosmetic composition according to Claim 7, characterised in that the size of the emulsified droplets varies between 0.5 and 50 µm.

10. Cosmetic composition according to Claim 7 or 9, characterised in that it is a silicone oil preferably chosen from polyalkylsiloxanes, polyalkylarylsiloxanes and mixtures thereof.

11. Cosmetic composition according to any one of Claims 1 to 10, characterised in that it contains 0.5 % to 8 % and preferably between 1.5 to 4.5 % of water-insoluble particles.

12. Cosmetic composition according to any one of the preceding claims, characterised in that it contains as anionic agent LESNa, as cosurface-active agent lauryl alcohol containing two moles of ethylene oxide and as electrolyte NaCl in a lauryl alcohol 2EO/LESNa mole ratio of between 2.1 and 4.3.

13. Cosmetic composition according to one of Claims 1 to 11, characterised in that it contains as anionic agent LESNa, as cosurface-active agent oleylbetaine and as electrolyte NaCl in an oleylbetaine/LESNa mole ratio of between 1.1 and 3.7.

14. Cosmetic composition according to one of Claims 1 to 11, characterised in that it contains as anionic agent a sodium lipoamino acid, as non-ionic agent a lauryl alcohol containing two ethoxy units in a lauryl alcohol 2EO/sodium lipoamino acid mole ratio varying between about 1.2 and 6.7.

15. Cosmetic composition according to any one of the preceding claims, characterised in that it is a shampoo, a shower gel, a hair lotion or an exfoliating gel.

16. Cosmetic composition according to any one of the preceding claims, characterised in that it does not contain organic solvents, in particular hydrocarbon-based solvents.

17. Process for preparing a cosmetic composition according to one of Claims 1 to 16, characterised in that it comprises:
- the mixing with stirring, in aqueous medium, of at least one anionic surface-active agent, one non-ionic and/or amphoteric cosurface-active agent and one electrolyte, the said surface-active agents being in amounts such that the resulting aqueous mixture has a pseudo-plastic behaviour with a yield point greater than or equal to 0.2 Pa and exhibits a lamellar phase structure enclosing spherulites in suspension,
- the addition to the said mixture of solid particles or of at least one oil with, in the case of the oil, the placing of the resulting aqueous mixture under vigorous stirring so as to emulsify the oil into fine droplets inside the aqueous mixture and
- the recovery of the said composition.

## Patentansprüche

1. Flüssige, wäßrige, kosmetische Zusammensetzung, die in Wasser nicht lösliche Teilchen in Suspension enthält,
dadurch gekennzeichnet, daß sie kein organisches Lösemittel enthält und darüberhinaus mindestens einen anionischen grenzflächenaktiven Stoff, einen zusätzlich enthaltenen grenzflächenaktiven Stoff nichtionischer oder amphoterer Art und einen Elektrolyten umfaßt, wobei die grenzflächenaktiven Stoffe in Mengen vorliegen, daß die Zusammensetzung ein pseudoplastisches Verhalten mit einem Fließpunkt gleich oder über 0,2 Pa zeigt und eine lamellare Phasenstruktur aufweist, die Sphärulite umfaßt, die die Teilchen stabil und homogen in Suspension halten können, wobei der Gehalt an Waschgrundlage unter 30% beträgt.

2. Kosmetische Zusammensetzung nach Anspruch 1,
dadurch gekennzeichnet, daß der anionische grenzflächenaktive Stoff vorzugsweise ausgewählt wird aus den Fettsäuresalzen; den Salzen von Mono- oder Dialkylsulfaten, Mono- oder Dialkylethersulfaten, Laurylethersulfaten, Alkylsulfonaten, Alkylarylsulfonaten, α-Olefinsulfonaten, alkalischen Alkylphosphaten, den Sulfonaten von Alkylphenolpolyglycolethern; den Salzen von Estern von Alkylsulfopolycarbonsäuren, den Salzen von Alkylsulfosuccinaten, Alkylethersulfocsuccinaten, Alkylisethionaten, Alkyltauraten, den Kondensationsprodukten von Fettsäuren mit den Oxy- und Aminoalkansulfonsäuren, den sulfatierten Derivaten von Fettsäure und Polyglycolen, den Alkanolamiden von sulfatierten Fettsäuren und den Salzen von Lipoaminosäuren.

3. Kosmetische Zusammensetzung nach Anspruch 1 oder 2,
dadurch gekennzeichnet, daß der zusätzlich enthaltene nichtionische grenzflächenaktive Stoff vorzugsweise ausgewählt wird aus den Zuckerglyceriden und den ethoxylierten Fettalkoholen und vorzugsweise unter jenen, die sich von Laurylalkohol, Cetylstearylalkohol, Stearylalkohol, Cetylalkohol und Oleocetylalkohol ableiten.

4. Kosmetische Zusammensetzung nach Anspruch 1, 2 oder 3,
dadurch gekennzeichnet, daß der zusätzlich enthaltene amphotere grenzflächenaktive Stoff ausgewählt wird unter den Betainen, wie Oleylbetain, und den Sulfobetainen vom Typ Cocoamidopropylhydroxysulfobetain und den Derivaten von Imidazolin, wie Cocoamphodiacetat.

5. Kosmetische Zusammensetzung nach einem der vorangegangenen Ansprüche,
dadurch gekennzeichnet, daß der Elektrolyt vorzugsweise Natriumchlorid oder Ammoniumchlorid ist.

6. Kosmetische Zusammensetzung nach einem der vorangegangenen Ansprüche,
dadurch gekennzeichnet, daß die in Wasser nicht löslichen Teilchen feste Teilchen und vorzugsweise aus Zinkpyrithion oder natürliche oder synthetische Schleifmaterialien sind.

7. Kosmetische Zusammensetzung nach einem der Ansprüche 1 bis 5,
dadurch gekennzeichnet, daß die in Wasser nicht löslichen Teilchen emulgierte Tröpfchen von mindestens einem pflanzlichen Öl, etherischen Öl und/oder Silikonöl sind.

8. Kosmetische Zusammensetzung nach Anspruch 6,
dadurch gekennzeichnet, daß die Größe der festen Teilchen zwischen ungefähr 1 und 600 µm und vorzugsweise zwischen 10 und 400 µm liegt.

9. Kosmetische Zusammensetzung nach Anspruch 7,
dadurch gekennzeichnet, daß die Größe der emulgierten Tröpfchen zwischen 0,5 und 50 µm variiert.

10. Kosmetische Zusammensetzung nach Anspruch 7 oder 9,
dadurch gekennzeichnet, daß es sich um ein Silikonöl, vorzugsweise ausgewählt aus den Polyalkylsiloxanen, den Polyalkylarylsiloxanen und deren Mischungen, handelt.

11. Kosmetische Zusammensetzung nach einem der Ansprüche 1 bis 10,
dadurch gekennzeichnet daß sie ungefähr 0,5% bis 8% und vorzugsweise 1,5 bis 4,5% in Wasser nicht lösliche Teilchen enthält.

12. Kosmetische Zusammensetzung nach einem der vorangegangenen Ansprüche,
dadurch gekennzeichnet, daß sie als anionisches Mittel LESNa, als zusätzlich enthaltenen grenzflächenaktiven Stoff Laurylalkohol mit zwei Molen Ethylenoxid und als Elektrolyt NaCl in einem Molverhältnis von Laurylalkohol-2OE/LESNa zwischen 2,1 und 4,3 enthält.

13. Kosmetische Zusammensetzung nach einem der Ansprüche 1 bis 11,
dadurch gekennzeichnet, daß sie als anionisches Mittel LESNa, als zusätzlich enthaltenen grenzflächenaktiven Stoff Oleylbetain und als Elektrolyt NaCl in einem Molverhältnis von Oleylbetain/LESNa zwischen 1,1 und 3,7 enthält.

14. Kosmetische Zusammensetzung nach einem der Ansprüche 1 bis 11,
dadurch gekennzeichnet, daß sie als anionisches Mittel ein Natriumsalz einer Lipoaminosäure, als nichtionisches Mittel einen Laurylalkohol mit zwei Ethoxyeinheiten in einem Molverhältnis von Laurylalkohol-2OE/Natriumsalz einer Lipoaminosäure zwischen 1,2 und 6,7 enthält.

15. Kosmetische Zusammensetzung nach einem der vorangegangenen Ansprüche,
dadurch gekennzeichnet, daß es sich um ein Shampoo, ein Duschgel, ein Haarwasser oder ein exfoliierendes Gel handelt.

16. Kosmetische Zusammensetzung nach einem der vorangegangenen Ansprüche,
dadurch gekennzeichnet, daß sie keine organische Lösemittel, insbesondere Kohlenwasserstofflösemittel, umfaßt.

17. Verfahren zur Herstellung einer kosmetischen Zusammensetzung nach einem der Ansprüche 1 bis 16,
dadurch gekennzeichnet, daß es umfaßt:
- das Mischen von mindestens einem anionischen grenzflächenaktiven Stoff, einem zusätzlichen nichtionischen und/oder amphoteren grenzflächenaktiven Stoff und einem Elektrolyten in wäßrigem Medium unter Bewegung, wobei die grenzflächenaktiven Stoffe in Mengen vorliegen, daß die resultierende wäßrige Mischung ein pseudoplastisches Verhalten mit einem Fließpunkt über oder gleich 0,2 Pa zeigt und eine lamellare Phasenstruktur aufweist, die Sphärulite in Suspension umfaßt,
- die Zugabe fester Teilchen oder mindestens eines Öls zu der Mischung, wobei im Falle eines Öls die resultierende wäßrige Mischung einer sehr starken Bewegung derart unterworfen wird, daß das Öl in feinen Tröpfchen innerhalb der wäßrigen Mischung emulgiert wird, und
- die Gewinnung der Zusammensetzung.
